# EUROPEAN PATENT APPLICATION

(11) **EP 3 143 934 A1**
(43) Date of publication of application: **22.03.2017**
(21) Application number: 15791963.0
(22) Date of filing: 14.05.2015
(51) Int. Cl.: A61B 6/00, A61B 6/14

(54) **X-RAY PHOTOGRAPHING APPARATUS ADOPTING WIRELESS POWER SUPPLY MANNER**

(30) Priority: 14.05.2014 KR 20140057847
(71) Applicant: Vatech Co. Ltd., Hwaseong-si, Gyeonggi-do 445-170 (KR); Vatech Ewoo Holdings Co., Ltd, Gyeonggi-do 445-170 (KR)
(72) Inventor: LIM, Hyung Keun, Hwaseong-si Gyeonggi-do 445-170 (KR); CHUN, Jin Pyo, Hwaseong-si Gyeonggi-do 445-170 (KR); CHOI, Sung Il, Hwaseong-si Gyeonggi-do 445-170 (KR)
(74) Representative: Markfort, Iris-Anne Lucie
(86) International application number: PCT/KR2015/004823
(87) International publication number: WO 2015/174746

(57) **Abstract**

The present invention relates to an X-ray photographing apparatus adopting a wireless power supply manner and, particularly, to an X-ray photographing apparatus adopting a wireless power supply manner, having no power cable for supplying power, and providing driving power for an X-ray photographing unit in a wireless manner.

## Description

### Technical Field

The present invention relates, generally, to an x-ray radiographing apparatus and, more particularly, to an x-ray radiographing apparatus wirelessly providing power to an x-ray radiographing part.

### Background Art

Generally, in dental clinics, a two-dimensional (2D) projection radiograph and a three-dimensional (3D) computed tomography radiograph of a patient are obtained by using an x-ray radiographing apparatus.

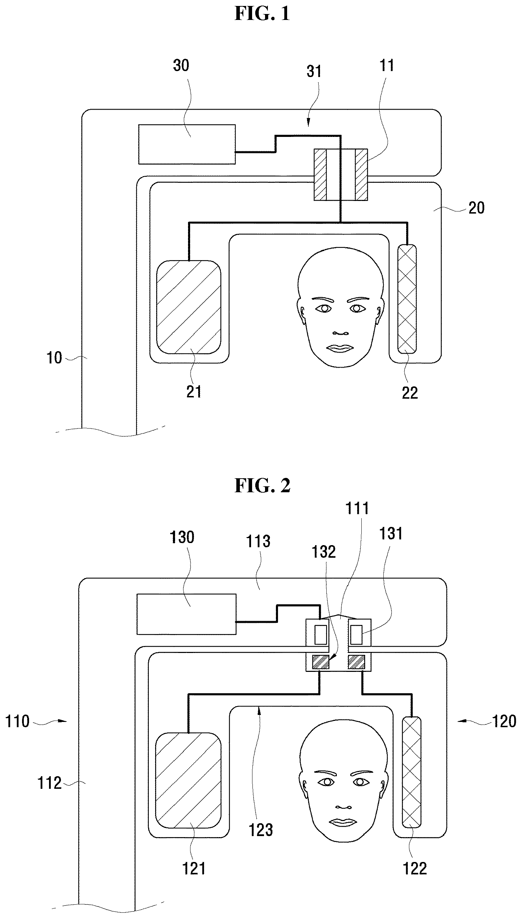

FIG.1 is a view showing a conventional x-ray radiographing apparatus. Referring to FIG. 1, the conventional x-ray radiographing apparatus includes a body 10 including a connection part 11 that is parallel to a rotation axis (not shown), an x-ray radiographing part 20 connected to the body 10 through the connection part 11 and capable of independently rotating, and a power supplying part 30 provided in the body 10 and providing driving power to the x-ray radiographing part 20. The x-ray radiographing part 20 includes an x-ray source 21 and a detector 22 that rotate about the rotation axis facing each other. The x-ray radiographing part 20 obtains multi-directional projection data of a subject by rotating around and radiographing the subject, and a 3D computed tomography radiograph of the subject is obtained by reconstructing the multi-directional projection data. The connection part 11 is provided with an empty inner part, and a power cable 31 of the power supplying part 30 is connected to the x-ray radiographing part 20 by passing through the connection part 11. In addition, a data cable for transmitting the obtained projection data of the x-ray radiographing part 20 may pass through the connection part 11 of the body 10.

Since the conventional x-ray radiographing apparatus is configured with the power cable 31 passing through the connection part 11 that is parallel to the rotation axis, the power cable 31 causes a load and generates interference when the x-ray radiographing part 20 rotates, and thus a rotation speed and a rotation range of the x-ray radiographing part 20 are limited. In addition, conventionally, the power cable is twisted by the rotation of x-ray radiographing part 20. The x-ray radiographing part 20 has to obtain multi-directional projection data to obtain an accurate 3D computed tomography radiograph; however, the rotation range of the x-ray radiographing part 20 is restricted by the power cable 31, and thus quality of a 3D computed tomography radiograph is decreased.

### Disclosure

### Technical Problem

As a result of efforts to improve the quality of a 3D computed tomography radiograph by preventing cable interference within a connection part of an x-ray radiographing part and expanding a rotation range thereof, the inventors have completed the present invention by developing an x-ray radiographing apparatus adopting a wireless power supplying manner.

Therefore, an object of the present invention is to provide an x-ray radiographing apparatus adopting a wireless power supplying manner to obtain a 3D computed tomography radiograph with high quality by expanding the rotation range of the x-ray radiographing part.

In addition, another object of the present invention is to provide an x-ray radiographing apparatus adopting a wireless power supplying manner capable of wirelessly supplying driving power to an x-ray radiographing part without using a power cable.

The present invention is not limited to the aforementioned objects, and other objects other than the aforementioned objects will be clearly comprehended to those skilled in the art from the following description.

### Technical Solution

In order to achieve the above object, according to one aspect of the present invention, there is provided an x-ray radiographing apparatus including: an x-ray source and a detector, both rotating around a subject interposed therebetween; a gantry with both the x-ray source and the detector installed therein; and a first power module provided in the gantry, wirelessly receiving power, and providing the received power to at least one of the x-ray source and the detector.

In a preferred embodiment, the x-ray source and the detector may be installed within the gantry to face each other.

In a preferred embodiment, the apparatus may further include a body connected to and supporting the gantry.

In a preferred embodiment, the first power module may be positioned within the gantry, and the apparatus may further include: a second power module provided within a body and wirelessly providing power to the first power module.

In a preferred embodiment, the body may further include: a column being perpendicular to a ground surface; and a lateral arm extending from the column in a lateral direction, wherein the gantry is connected to the body through both the lateral arm and a connection part, and the first power module and the second power module are provided in the gantry and in the lateral arm, respectively.

In a preferred embodiment, the second power module may wirelessly provide power to the first power module, the second power module and first power module being placed at positions closest to each other, or within an effective distance while operating the X-ray radiographing apparatus.

In a preferred embodiment, the body may further include: a column being perpendicular to a ground surface; and a lateral arm extending from the column in a lateral direction, wherein the gantry is connected to the body through both the lateral arm and a connection part, and the first power module and the second power module are provided in the gantry and in the lateral arm, respectively.

In a preferred embodiment, the first power module may be provided on one side of the x-ray source or the detector of the x-ray radiographing part.

In a preferred embodiment, the first power module and the second power module exchange the power wirelessly at closest positions or in an effective distance while operating the x-ray radiographing apparatus.

In a preferred embodiment, the first power module may include a chargeable battery.

### Advantageous Effects

The present invention has outstanding effects as follows.

First, according to the x-ray radiographing apparatus according to an embodiment of the present invention, the second power module wirelessly provides power to the first power module disposed in the x-ray radiographing part, and thus an additional cable used for connecting the second power module and the first power module is not required, thus the rotation range of the x-ray radiographing part is also expanded.

In addition, since the rotation range of the x-ray radiographing part is expanded, the x-ray radiographing apparatus according to the present invention improves quality of a 3D computed tomography radiograph by obtaining multi-directional projection data of a subject.

### Description of Drawings

FIG.1 is a view showing a conventional x-ray radiographing apparatus.
FIG. 2 is a cross sectional view of an x-ray radiographing apparatus according to a first embodiment of the present invention.
FIG. 3 is a top plan view of the x-ray radiographing apparatus according to the first embodiment of the present invention.
FIG. 4 is a cross sectional view of an x-ray radiographing apparatus according to a second embodiment of the present invention.
FIG. 5 is a top plan view of the x-ray radiographing apparatus according to the second embodiment of the present invention.
FIG. 6 is a cross sectional view showing another layout structure of a power supplying part according to the second embodiment of the present invention.
FIG. 7 is a cross sectional view showing a layout structure of a power supplying part according to a third embodiment of the present invention.

### Description of Reference Numerals in the Drawings

110: body 111: connection part
112: column 113: lateral arm
120: x-ray radiographing part 121: x-ray source

### Best Mode

As the terms used in the present invention, general terms that are widely used at present are selected, but terms that are arbitrarily selected by the applicant are used in particular cases. In this case, these terms should be interpreted not as dictionary definition thereof but the meaning described in the detailed description for implementing the invention or the meaning of the terms.

Hereinafter, a technical configuration of the present invention will be described in detail with reference to preferred embodiments illustrated in the accompanying drawings.

However, the present invention is not limited to the embodiments described herein, but may also be embodied in other forms. Throughout the specification, the same reference numerals designate the same components.

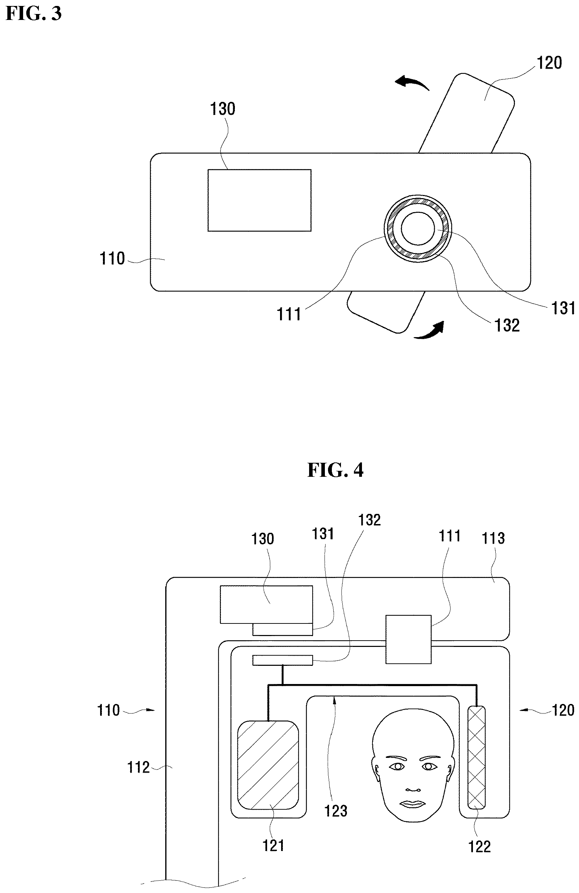

FIG. 2 is a cross sectional view of an x-ray radiographing apparatus according to a first embodiment of the present invention, and FIG. 3 is a top plan view of the x-ray radiographing apparatus according to the first embodiment of the present invention.

Referring to FIGS. 2 and 3, the x-ray radiographing apparatus according to the first embodiment of the present invention includes a body 110, an x-ray radiographing part 120, and power supplying parts 130, 131, and 132.

The body 110 is used for supporting the x-ray radiographing part 120 that will be described later such that the radiographing part 120 is capable of rotating. The body 110 includes: a connection part 111 that is provided in a predetermined position and is parallel to a rotating axis, a column 112 that is perpendicular to a ground surface, and a lateral arm 113 that extends from the column 112 in a lateral direction and is parallel to the ground surface. The body 110 is configured to be capable of being extended or contracted in vertical directions such that a height of the body 110 is adjusted. The body 110 may be installed and fixed to a wall of a building. The body 110 is provided with a main power supplying part 130 that is connected to commercial power. The connection part 111 is provided in a predetermined position of the lateral arm 113. Although it is not shown, the x-ray radiographing apparatus may further include a moving part that is provided in a gantry 123 or within the body 110 (preferably, within the connection part 111) such that the x-ray radiographing part 120 is capable of moving along a subject according to a predetermined path.

The x-ray radiographing part 120 is used for radiographing a subject that is a patient, and may be configured to include an x-ray source 121 irradiating x-rays to the subject, a detector 122 obtaining projection data by detecting the x-rays passing through the subject, and a gantry 123 including an x-ray source installing part and a detector installing part such that the x-ray source 121 and the detector 122 face each other. The gantry 123 is connected to the body 110 through the connection part 111. It is preferable to connect the gantry 123 to the lateral arm 113. The x-ray source 121 and the detector 122 are disposed to face each other. The x-ray source 121 and the detector 122 rotate around the subject, and the x-ray radiographing part 120 obtains projection data of the subject from various directions. A 3-dimensinal radiograph may be obtained by reconstructing the obtained multi-directional projected data.

The main power supplying part 130 receives commercial power supplied from outside, converts the commercial power to proper driving power for operating the x-ray radiographing part 120, and provides the converted driving power to the x-ray radiographing part 120. Although it is not shown, the main power supplying part 130 may include a filter circuit that filters out electromagnetic waves and a converter circuit that converts AC power to DC power.

The body 110 includes a second power module 131 that receives power from the main power supplying part 130 and wirelessly provides the received power to the x-ray radiographing part 120 from the main power supplying part 130. The x-ray radiographing part 120 includes a first power module 132 that wirelessly receives power from the second power module 131 and provides the received power to at least one of the x-ray source 121 and the detector 122 of the x-ray radiographing part 120. The second power module 131 and the first power module 132 may use a magnetic induction method or a magnetic resonance method that transfers near field magnetic energy. For this, the second power module 131 is configured to include a first coil and the first power module 132 is configured to include a second coil such that the second power module 131 provides power to the first power module 132 by using magnetic resonance or magnetic induction.

In the first embodiment of the present invention, the second power module 131 and the first power module 132 are respectively disposed around the connection part 111 of body 110 and are spaced apart from each other in a vertical direction. In other words, the second power module 131 is disposed in the body 110 and around the connection part 111, and the first power module 132 is disposed in the gantry 123 and around the connection part 111 such that the second power module 131 and the first power module 132 face each other. The second power module 131 and the first power module 132 are configured to be spaced apart in a vertical direction; however, it is preferable to dispose the second power module 131 and the first power module 132 inside the body 110 and inside the x-ray radiographing part 120, respectively, such that the second power module 131 and the first power module 132 are placed at positions closest to each other. Since the second power module 131 and the first power module 132 are configured in a wireless connection, and not in a wired connection, driving power for operating the x-ray radiographing part 120 may be provided without generating limit or interference on a rotation range of the x-ray radiographing part 120. In addition, since the connection part 111 does not include an additional power cable, the x-ray radiographing part 120 is able to rotate 360 degrees. The x-ray radiographing part 120 is able to obtain projection data of 360 degrees without any limitation of the rotation range, and thus may obtain an improved a three-dimensional (3D) computed tomography radiograph that is a final result.

A process to provide power from the second power module 131 to the first power module 132 will be described. First, in case of the magnetic induction method, when commercial power is applied to the main power supplying part 130, current flows in the first coil of the second power module 131; then, an induced current is generated in the second coil of the first power module 132 by the current flowing in the first coil of the second power module 131. The induced current generated in the second coil is rectified and smoothed, and the rectified and smoothed current is able to be used as the driving power and is provided to the x-ray radiographing part 120. In addition, in case of the magnetic resonance method, when an electric signal having a resonance frequency that satisfies a resonance condition of external power is applied to the first coil of the second power module 131, magnetic flux is generated in the first coil and the generated magnetic flux is transferred to the second coil of the first power module 132. Then, the second coil of the first power module 132 resonates and generates induced electromotive force. Further, the generated induced electromotive force of the second coil is used as the driving power and is provided to the x-ray radiographing part 120.

### Mode for Invention

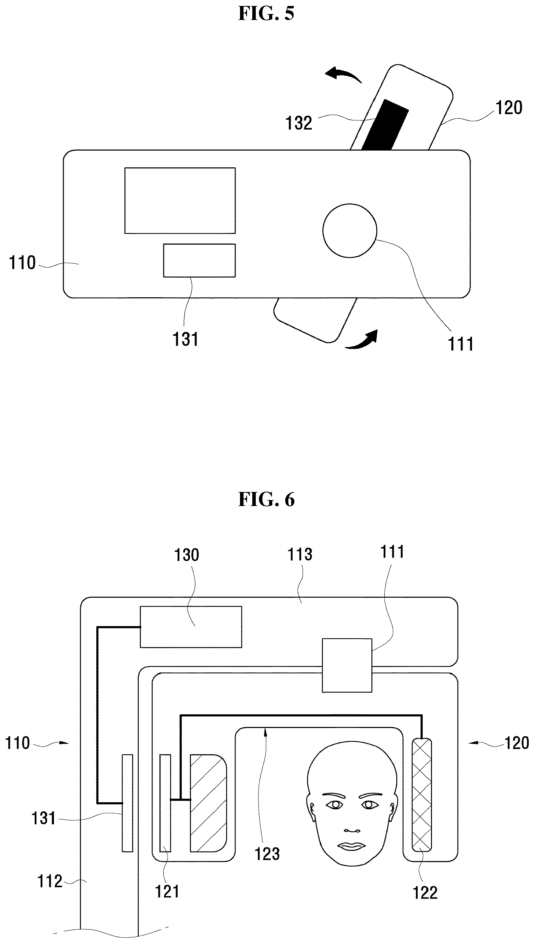

FIG. 4 is a cross sectional view of an x-ray radiographing apparatus according to a second embodiment of the present invention, FIG. 5 is a top plan view of the x-ray radiographing apparatus according to the second embodiment of the present invention, and FIG. 6 is a cross sectional view showing another layout structure of a power supplying part according to the second embodiment of the present invention.

First, referring to FIGS. 4 to 6, the x-ray radiographing apparatus according to the second embodiment of the present invention includes a body 110, an x-ray radiographing part 120, and power supplying parts 130, 131, and 132.

The body 110 is used for support and rotation of the x-ray radiographing part 120 and is provided with a connection part 111 in a predetermined position thereof. In addition, the body 110 includes a main power supplying part 130 that is connected to commercial power. The body 110 is configured to include a column 112 that is perpendicular to a ground surface and a lateral arm 113 with the connection part 111 provided therein and extends from the column 112 in a lateral direction and is parallel to the ground surface. The x-ray radiographing part 120 is used for radiographing a subject that is a patient, and may be configured to include an x-ray source 121 irradiating x-rays to the subject and a detector 122 obtaining projection data by detecting the x-rays passing through the subject, and a gantry 123 including an x-ray source installing part and a detector installing part such that the x-ray source 121 and the detector 122 face each other. The x-ray radiographing part 120 is configured to rotate by being connected to the connection part 111 of the body 110, and to obtain projection data by rotating around the subject. In addition, a 3D computed tomography radiograph may be obtained by reconstructing multi-directional projected data. The body 110 and the x-ray radiographing part 120 may be configured substantially the same as those of the first embodiment. Although it is not shown, the x-ray radiographing apparatus may further include a moving part that is provided in the gantry 123 or within the body 110 (preferably, within the connection part 111) such that the x-ray radiographing part 120 is capable of moving along a subject according to a predetermined path.

The main power supplying part 130 is the same as that of the first embodiment in that the main power supplying part 130 receives commercial power and converts the commercial power to proper driving power for operating the x-ray radiographing part 120. However, a second power module 131 and a first power module 132 are differently disposed with respect to the first embodiment. In one embodiment, as shown in FIG. 4, the second power module 131 may be disposed within the lateral arm 113 of the body 110 but spaced apart from the connection part 111, and the first power module 132 may be disposed within the gantry 123 of the x-ray radiographing part 120. In another embodiment, as shown in FIG. 6, the second power module 131 may be disposed within the column 112 of the body 110 and the first power module 132 may be disposed in one side of the x-ray source 121 of the x-ray radiographing part 120. Alternatively, the first power module 132 may be disposed in one side of detector 122 of the x-ray radiographing part 120. In other words, the driving power for operating the x-ray radiographing part 120 may be wirelessly provided to the x-ray radiographing part 120 without disposing the second power module 131 and the first power module 132 close to the connection part 111 of the body 110.

When the x-ray radiographing part 120 is placed at a radiograph starting point or a radiograph standby point, the first power module 132 according to the second embodiment of the present invention receives power transmitted from the second power module 131 by facing the second power module 131 in the closest proximity as shown in FIG. 4 or FIG. 6.

In addition, the first power module 132 may receive power transmitted from the second power module 131 when the second power module 131 and the first power module 132 are placed at positions closest to each other, or within an effective distance while radiographing.

Herein, it is preferable to configure the x-ray radiographing part 120 to be capable of charging the receiving the power transmitted from the second power module 131 or to include an additional chargeable battery. In other words, the first power module 132 is capable of wirelessly receiving power form the second power module 131 by being disposed close to the second power module 131 while radiographing and during standby. In the second embodiment, the second power module 131 and the first power module 132 are not placed as the first embodiment, which is restricted to nearby the connection part 111 of the body 110. Accordingly, the second power module 131 and the first power module 132 are easy to manufacture without causing rotation interference of the x-ray radiographing part 120.

In addition, in a conventional x-ray radiographing apparatus, the x-ray radiographing part 120 waits at the standby point for a long time, and thus the x-ray radiographing apparatus has enough time to be charged with driving power.

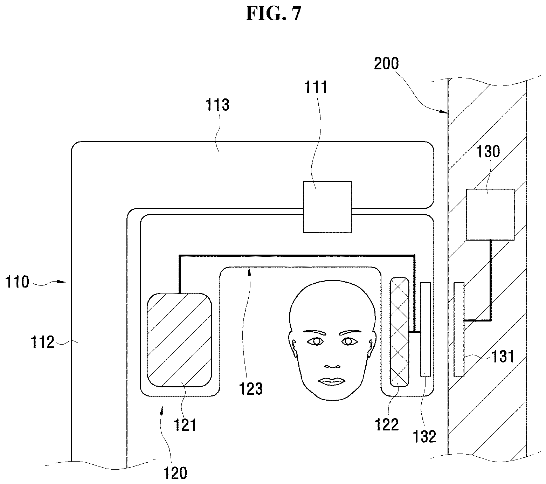

FIG. 7 is a sectional view showing a layout structure of a power supplying part according to a third embodiment of the present invention.

Referring to FIG. 7, an x-ray radiographing apparatus according to the third embodiment includes a body 110, an x-ray radiographing part 120, and power supplying parts 130, 131, and 132.

The body 110 is used for supporting the x-ray radiographing part 120 such that the radiographing part 120 is capable of rotating. The body 110 is configured to include a connection part 111 connected to the x-ray radiographing part 120, a column 112 that is perpendicular to a ground surface, and a lateral arm 113 with the connection part 111 provided therein, the lateral arm 113 extending from the column 122 in a lateral direction, and being parallel to the ground surface.

The x-ray radiographing part 120 is used for radiographing a subject that is a patient, and may be configured to include an x-ray source 121 irradiating x-rays to the subject and a detector 122 obtaining projection data by detecting the x-rays passing through the subject, and a gantry 123 including a source installing part and a detector installing part such that the source 121 and the detector 122 face each other. The body 110 and the x-ray radiographing part 120 may be configured substantially the same as those of the first or the second embodiment. Although it is not shown, the x-ray radiographing apparatus may further include a moving part that is provided in the gantry 123 or within the body 110 (preferably, within the connection part 111) such that the x-ray radiographing part 120 is capable of moving along a subject according to a predetermined path.

A main power supplying part 130 receives commercial power from outside, converts the commercial power to proper driving power for operating the x-ray radiographing part 120, and provides the converted power to the x-ray radiographing part 120. Although it is not shown, the main power supplying part 130 may include a filter circuit that filters out electromagnetic waves and a converter circuit that converts AC power to DC power.

In the third embodiment, a second power module 131 is not disposed inside the body 110 or the x-ray radiographing part 120 and is disposed in a predetermined position outside of the x-ray radiographing apparatus. A first power module 132 is disposed in an outer side of the x-ray source 121 or the detector 122 of the x-ray radiographing part 120. Herein, the predetermined position outside of the x-ray radiographing apparatus of the second power module 131 may be, for example, a surface of a wall. In other words, although the second power module 131 is not provided inside the body 110 or the x-ray radiographing part 120, driving power for operations the x-ray radiographing part 120 is provided by disposing the second power module 131 with the first power module 132 within a predetermined effective distance.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### Industrial Applicability

The present invention is applicable to a medical x-ray radiographing apparatus, more particularly, to an x-ray radiographing apparatus for dental clinics.

## Claims

1. An x-ray radiographing apparatus comprising:
an x-ray source and a detector, both rotating around a subject interposed therebetween;
a gantry including the x-ray source and the detector therein; and
a first power module provided in the gantry, wirelessly receiving power, and providing the received power to at least one of the x-ray source and the detector.

2. The x-ray radiographing apparatus of claim 1, wherein the x-ray source and the detector are installed within the gantry to face each other.

3. The x-ray radiographing apparatus of claim 2, further comprising: a body connected to and supporting the gantry.

4. The x-ray radiographing apparatus of any one of claims 1 to 3, wherein the first power module is positioned within the gantry, and
the apparatus further comprises: a second power module provided within a body and wirelessly providing power to the first power module.

5. The x-ray radiographing apparatus of claim 4, wherein the body further includes: a column being perpendicular to a ground surface; and a lateral arm extending from the column in a lateral direction, wherein the gantry is connected to the body through both the lateral arm and a connection part, and the first power module and the second power module are provided in the gantry and in the lateral arm, respectively.

6. The x-ray radiographing apparatus of claim 5, wherein the second power module wirelessly provides power to the first power module, the second power module and first power module being placed at positions closest to each other, or within an effective distance while operating the x-ray radiographing apparatus.

7. The x-ray radiographing apparatus of claim 4, wherein the body further includes: a column being perpendicular to a ground surface; and a lateral arm extending from the column in a lateral direction, wherein the gantry is connected to the body through both the lateral arm and a connection part, and the first power module and the second power module are provided in the gantry and in the lateral arm, respectively.

8. The x-ray radiographing apparatus of claim 7, wherein the first power module is provided on one side of the x-ray source or the detector of the x-ray radiographing part.

9. The x-ray radiographing apparatus of claim 8, the first power module and the second power module exchange the power wirelessly at closest positions or in an effective distance while operating the x-ray radiographing apparatus.

10. The x-ray radiographing apparatus of claim 1, wherein the first power module includes: a chargeable battery.
